(19) Europäisches Patentamt / European Patent Office / Office européen des brevets

(11) **EP 3 894 466 B1**

(12) **FASCICULE DE BREVET EUROPEEN**

(45) Date de publication et mention de la délivrance du brevet:
**04.01.2023 Bulletin 2023/01**

(21) Numéro de dépôt: **19868197.5**

(22) Date de dépôt: **29.11.2019**

(51) Classification Internationale des Brevets (IPC):
*C08K 3/16* $^{(2006.01)}$   *C08K 5/053* $^{(2006.01)}$
*C08L 29/04* $^{(2006.01)}$   *A61B 5/266* $^{(2021.01)}$
*C08K 3/04* $^{(2006.01)}$

(52) Classification Coopérative des Brevets (CPC):
(C-Sets disponibles)
**C08K 3/16; C08K 5/053;** A61B 5/25;
A61B 2562/0209; C08K 3/04          (Cont.)

(86) Numéro de dépôt international:
**PCT/FR2019/052866**

(87) Numéro de publication internationale:
**WO 2020/120865 (18.06.2020 Gazette 2020/25)**

(54) **COMPOSITION POLYMÉRIQUE CONDUCTRICE**

LEITFÄHIGE POLYMERZUSAMMENSETZUNG

POLYMERIC CONDUCTIVE COMPOSITION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.12.2018 FR 1872804**

(43) Date de publication de la demande:
**20.10.2021 Bulletin 2021/42**

(73) Titulaire: **Conscious Labs SAS 87000 Limoges (FR)**

(72) Inventeurs:
• **ARMAND, Michel 75014 PARIS (FR)**
• **ZOU, Shiyu 67000 STRASBOURG (FR)**
• **DAUGUET, Julien 75015 PARIS (FR)**

(74) Mandataire: **Gauchet, Fabien Roland Brandon IP 64, rue Tiquetonne 75002 Paris (FR)**

(56) Documents cités:
**EP-A2- 0 588 238      FR-A1- 2 464 077
US-A- 4 692 273      US-A- 5 686 516
US-A1- 2018 192 906   US-A1- 2018 256 105**

(52) Classification Coopérative des Brevets (CPC):
(Cont.)

C-Sets
**C08K 3/16, C08L 29/04;**
**C08K 5/053, C08L 29/04;**
C08K 3/04, C08L 29/04

**Description**

La présente invention est relative à une composition polymérique adaptée à une utilisation comme matériau conducteur dans des électrodes de mesure des signaux électro-physiologiques.

Les signaux électro-physiologiques sont le résultat de l'activité électrochimique des cellules vivantes, qui génère des différences de potentiel électrique, communément dénommées « biopotentiels ».

La mesure des signaux de biopotentiel générés par l'activité électrique des cellules est une pratique courante dans le domaine médical, par exemple dans le cadre de l'électrocardiographie (ECG) pour l'étude de la fonction cardiaque, ou l'électroencéphalographie (EEG) pour celle de l'activité cérébrale. Pour des explorations non-invasives, cette activité électrique est mesurée à l'aide d'électrodes positionnées en surface de la peau ou du cuir chevelu, aux endroits du corps choisis en fonction du type de mesure à effectuer.

Ainsi par exemple, l'EEG consiste à mesurer l'activité électrique du cerveau en mesurant les différences de potentiel électrique entre des électrodes placées à la surface du cuir chevelu.

Les électrodes servent de transducteurs pour transformer le courant ionique généré par l'activité cellulaire en courant électronique.

Classiquement, on utilise des électrodes constituées le plus souvent d'une plaque d'argent recouverte d'un film de chlorure d'argent (électrodes Ag/AgCl). Ces électrodes, qui sont utilisées avec un gel aqueux conducteur appliqué entre la peau et l'électrode, sont appelées « électrodes humides ».

L'utilisation de gel conducteur permet d'abaisser l'impédance peau-électrode en hydratant la couche cornée de l'épiderme ce qui facilite la transduction du courant ionique en courant électronique. En outre, le gel permet également de maintenir un meilleur contact entre la peau et l'électrode en cas de mouvements du sujet, ce qui limite les perturbations qui peuvent résulter de ce mouvement.

Cependant, les électrodes humides présentent divers inconvénients qui sont bien connus. En premier lieu, préalablement au placement des électrodes il est nécessaire classiquement, de préparer les zones du cuir chevelu choisies par rasage suivi d'une abrasion légère et d'un nettoyage à l'alcool pour amincir la couche cornée. Ces opérations demandent du temps et l'intervention d'un opérateur extérieur. En outre, les produits abrasifs utilisés pour la préparation ainsi que le gel conducteur laissent des résidus sur le cuir chevelu et les cheveux et peuvent même dans certains cas provoquer des irritations chez des sujets à peau sensible. Bien que convenant à l'utilisation pour des mesures EEG effectuées à l'hôpital, en cabinet médical, ou en laboratoire de recherche, elles ne sont pas adaptées à une utilisation sur le terrain ou dans un dispositif EEG qui s'adresserait au grand public.

Il a été proposé d'utiliser des électrodes dites « sèches », qui présentent l'avantage de ne pas nécessiter d'utilisation de gel (pour revue cf. Lopez-Gordo, et al., Sensors 2014, 14(7), 12847-12870).

La plupart des électrodes sèches sont métalliques (principalement Ag/AgCl) et rigides et présentent des micro-picots conducteurs. L'absence de gel est compensée par le fait que ces picots peuvent pénétrer la couche cornée et sont ainsi en mesure de capter directement les courants ioniques. Ces électrodes ont une faible impédance, mais leur rigidité et la présence des picots les rendent inconfortables.

Plus récemment, toujours afin d'améliorer le confort et la facilité d'utilisation de dispositifs EEG à destination notamment du grand public, des électrodes non métalliques, et même souples sont apparues. Notamment, des électrodes en polymère chargé par des particules d'un matériau conducteur électronique ont été réalisées. Si ces électrodes sont clairement plus confortables que les électrodes métalliques et peuvent être de relativement bons conducteurs électroniques, elles sont de très mauvais conducteurs ioniques et présentent des performances de mesure très dégradées comparées aux électrodes Ag/AgCl + gel électrolytique, ou même à des électrodes sèches rigides métalliques. Une façon de compenser cette très faible conductivité ionique consiste à les rendre actives, c'est-à-dire d'ajouter un circuit de pré-amplification à la source de la mesure. Ceci permet d'artificiellement amplifier le faible courant mesuré et donc de réduire l'impédance résultante, rendant la mesure plus robuste et moins sensible au bruit inhérent à l'EEG. Cependant cette solution n'apporte qu'une faible amélioration, l'électrode reste un très faible conducteur ionique. De plus, l'ajout d'un circuit actif augmente le coût et la complexité de mise en place (câbles supplémentaires, rigidité augmentée du câblage) et n'est notamment pas du tout optimal dans le cas d'un casque comportant un grand nombre de capteurs, ou dans le cas d'une recherche de coût minimal (dispositif grand public). Le remplacement éventuel de l'électrode est également plus complexe et plus cher. FR 2 464 077 décrit des électrodes biomédicales sèches à base de polymère hydrophile non ionique. Des plastifiants tels que le glycérol peuvent être ajoutés. Pas de sel est ajouté.

La présente invention a pour but de fournir des électrodes sèches comprenant ou constituées par un polymère souple, et ne présentant pas les inconvénients des électrodes sèches connues dans l'art antérieur, notamment ne nécessitant pas l'ajout d'un circuit de pré-amplification.

Dans ce but, la présente invention fournit un matériau polymérique spécifiquement adapté à la fabrication de telles électrodes.

La présente invention a pour objet une composition polymérique conductrice ionique définie par la formule générale suivante :

$$(PH)x + (SOH)y + z(MCl) \; ;$$

dans laquelle :

- PH représente un polymère contenant des fonctions protiques constituées par des groupes hydroxyle
- SOH représente un polyol plastifiant de masse moléculaire supérieure ou égale à 75 g/mol et inférieure ou égale à 250 g/mol, sous forme de molécules discrètes ;
- MCl représente du chlorure de sodium ou de potassium (M= Na ou K) ;
- $0,3 \le x/y \le 3$, x représentant la quantité en poids du polymère PH, et y la quantité en poids du polyol SOH ;
- $0,5 \% \le z \le 15\%$, z représentant le pourcentage en poids de MCl par rapport au polyol SOH.

Cette composition polymérique se présente sous la forme d'un élastomère souple et sec au toucher, n'exsudant pas le composant SOH.

Le polymère PH peut ainsi être notamment un produit d'hydrolyse du poly(acétate de vinyle), de degré de saponification supérieur ou égal à 60% et inférieur ou égal à 100%, de préférence supérieur ou égal à 80% et inférieur ou égal à 100%, et de masse moléculaire moyenne ($M_w$) supérieure ou égale à $5.10^4$ et inférieure ou égale à $2.10^6$ daltons, de préférence supérieure ou égale à $1.10^5$ et inférieure ou égale à $1.10^6$ - daltons. De tels polymères sont connus sous le nom d'alcools poly(vinylique)s (abrégé ci-après en PVA).

Le polyol plastifiant SOH peut par exemple être choisi parmi le glycérol, le propylène glycol, le dipropylène glycol ou leurs mélanges. De préférence, SOH est le glycérol ou le dipropylène glycol, et de manière tout à fait préférée SOH est le glycérol.

Préférentiellement :

- le rapport x/y est tel que $0,50 \le x/y \le 1,00$, en particulier $0,60 \le x/y \le 0,80$, de préférence $0,62 \le x/y \le 0,75$, avantageusement $0,63 \le x/y \le 0,71$, et de manière particulièrement préférée $0,64 \le x/y \le 0,69$.
- le pourcentage z est tel que $1 \% \le z \le 15 \%$, avantageusement $4 \% \le z \le 6 \%$, et de préférence $4,5 \% \le z \le 5,5 \%$.

Les propriétés de conductivité ionique de la composition polymérique conforme à l'invention la rendent particulièrement appropriée à l'utilisation dans des électrodes de mesure de signaux électro-physiologiques, notamment dans des électrodes destinées à l'EEG.

Il est possible de conférer en outre à une composition polymérique conductrice ionique conforme à l'invention une bonne conductivité électronique en lui adjoignant un ou plusieurs additif(s) carboné(s) particulaire(s) conducteur(s) électronique(s), et notamment, à titre d'exemples non limitatifs, un ou plusieurs additif(s) carboné(s), tels que les graphites, les fibres de graphite, les poudres de noir de carbone, les fibres et nanotubes de carbone.

En conséquence, selon un mode de réalisation préféré d'une composition polymérique conforme à l'invention, elle contient en outre une charge carbonée particulaire électro-conductrice.

Avantageusement, le pourcentage en poids de la charge conductrice par rapport au polymère PH est de 5% à 60%, de préférence de 10% à 50%, avantageusement de 20 % à 50%.

Pour améliorer encore les propriétés conductrices d'une composition polymérique conforme à l'invention, on peut lui adjoindre en outre un couple rédox permettant la transition de la conductivité ionique à la conductivité électronique. Avantageusement, le couple rédox est un mélange Ag/AgCl, qui peut être ajouté sous forme de poudre aux autres constituants, à raison de 1 % à 8% en poids par rapport au polymère PH.

La présente invention a également pour objet :

- une électrode de mesure d'un signal électro-physiologique comprenant une composition polymérique conforme à l'invention ;
- un dispositif de mesure d'un signal électro-physiologique comprenant une ou plusieurs électrodes conformes à l'invention.

La présente invention sera mieux comprise à l'aide du complément de description qui va suivre, qui se réfère à des exemples non-limitatifs décrivant la préparation et les propriétés de compositions polymérique conductrices et d'électrodes conformes à l'invention.

## EXEMPLE 1: PRÉPARATION DE COMPOSITIONS POLYMÉRIQUES CONDUCTRICES

Des mélanges en différentes proportions d'alcool polyvinylique, de glycérol, et de chlorure de sodium ont été réalisés.

L'alcool polyvinylique (M$_w$ ~195,000, *SIGMA-ALDRICH*), le glycérol (qualité réactif, ≥99.0% (GC), *SIGMA-ALDRICH*) et le chlorure de sodium sont pesés dans un bécher et dissous dans l'eau déminéralisée (rapport en poids PVA/eau = 1:10) en chauffant à environ 60°C pendant environ 1 heure, sous agitation à l'aide d'un barreau aimanté.

Dans une autre série d'expérimentations une composition polymérique chargée de poudre de graphite a été préparée, selon le protocole décrit ci-dessus, à ceci près que la poudre de graphite est ajoutée aux autres constituants préalablement à la dissolution. Différentes concentrations de poudre de graphite (Graphit GNP 12, pureté 99,5%, taille des particules 16-63$\mu$m) ont été testées.

## EXEMPLE 2: FABRICATION D'ÉLECTRODES

Lorsque la solution de composition polymérique a atteint une viscosité suffisante pour stopper la rotation du barreau aimanté, elle peut être utilisée pour la fabrication des électrodes.

Électrode plane : Cette électrode est préparée en plongeant pendant quelques instants une électrode d'or passive Gold Cup (OpenBCI) dans la solution de composition polymérique. Une fois l'électrode d'or enrobée de composition, on laisse sécher l'ensemble à l'air libre et à température ambiante pendant au moins 3 jours environ.

Électrode à picots : Un moule d'électrode avec des picots est fabriqué par impression 3D (matériau : acide polylactique). Ce moule est rempli avec la solution de composition polymérique et une électrode Gold Cup y est ensuite plongée. On laisse sécher l'ensemble à l'air libre et à température ambiante pendant au moins 1 semaine, avant de procéder au démoulage.

## EXEMPLE 3 : ESSAI DES PROPRIÉTÉS CONDUCTRICES DES ÉLECTRODES

### 1) Mesure du rapport signal/bruit

Le rapport signal/bruit (ou SNR pour signal-to-noise ratio) est un rapport de puissance des signaux et du bruit. C'est une mesure de fidélité de transmission des signaux.

Pour le déterminer, les électrodes fabriquées comme décrit ci-dessus ont été testées pour mesurer l'activité $\alpha$ (8 - 12 Hz) par EEG.

*Montage EEG :*

3 électrodes ont été utilisées pour chaque mesure : une électrode de mesure, une électrode de référence, et une électrode de polarisation (bias).

Dans le cas des électrodes planes, toutes les électrodes ont été placées sur des zones de peau glabre, à savoir sur le front pour l'électrode de mesure, et sur le lobe de chaque oreille pour l'électrode de référence et l'électrode de polarisation.

Dans le cas d'utilisation d'électrodes à picots, l'électrode de mesure est placée au sommet du crâne (vertex : position Cz selon le Système International 10-20) et les électrodes de référence et de polarisation sur le lobe de chaque oreille.

Les mesures sont effectuées sur 2 sessions de 2 minutes chacune, 1 minute yeux ouverts, et 1 minute yeux fermés (la puissance dans la bande $\alpha$ augmentant lorsque les yeux sont fermés).

*Calcul du rapport signal/bruit :*

La puissance relative d'activité d'alpha est calculée à l'aide de la formule suivante :

$$\text{Puissance relative} = \text{Puissance d'alpha}(8 - 12\,\text{Hz})/\text{Puissance totale du signal }(1 - 60\,\text{Hz})$$

Le rapport signal/bruit (SNR) est ensuite calculé comme décrit par Tautan et al. (Proc. 7th International Conference on Biomedical Electronics and Devices ; Biodevices 2014).

Plus le SNR est élevé, plus l'électrode est sensible.

*Influence du rapport PVA:glycérol sur le rapport signal/bruit*

Le SNR est calculé comme décrit ci-dessus, pour différentes proportions PVA: glycérol, à concentration de NaCl constante de 5 % en poids par rapport au glycérol. La quantité de PVA est utilisée comme référence. La proportion théorique de glycérol augmente de 0,66 à 1,75.

Les résultats sont illustrés par le Tableau 1 ci-dessous :

[Table 1]

| PVA: Glycérol | SNR | SNR moyen |
|---|---|---|
| 1:0,66 | / | / |
| 1:1,03 | 4,1083 | |
| 1:1,01 | 5,3627 | 5,1026 |
| 1:1,01 | 6,0968 | |
| 1:1,55 | 5,3691 | |
| 1:1,52 | 4,9488 | 5,4716 |
| 1:1,53 | 6,0968 | |
| 1:1,75 | 5,2216 | / |

Pour les plus faibles quantités de glycérol (rapport PVA: glycérol <1), aucun signal EEG n'a pu être détecté. En augmentant la proportion de glycérol, le SNR augmente, mais diminue à nouveau pour la quantité de glycérol la plus importante. De plus, dans le cas du rapport PVA: glycérol 1 : 1,75 glycérol, on observe une exsudation du glycérol après séchage, rendant l'électrode impropre à l'utilisation.

*Influence de la concentration en NaCl sur le rapport signal/bruit*

L'influence de la concentration de NaCl a ensuite été testée. Les résultats sont illustrés dans le Tableau 2 ci-dessous. Les % de NaCl indiqués sont des pourcentages en poids par rapport au glycérol (la concentration de saturation du NaCl dans le glycérol est de 7,5%).

[Table 2]

| PVA : Glycérol | NaCl (g) | [NaCl] (% p.p.) | SNR | SNR moyen |
|---|---|---|---|---|
| 1:1,52 | 0,04 | 3,15 | / | / |
| 1:1,55 | 0,05 | 4,90 | 5,3691 | |
| 1:1,52 | 0,10 | 5,00 | 4,9488 | 5,4716 |
| 1:1,53 | 0,08 | 4,95 | 6,0968 | |
| 1:1,46 | 0,08 | 6,84 | 4,0919 | |
| 1:1,51 | 0,08 | 6,96 | 3,4859 | 3,6745 |
| 1:1,46 | 0,07 | 6,86 | 3,4457 | |
| 1:1,52 | 0,12 | 9,76 | 6,4631 | |
| 1:1,48 | 0,10 | 9,80 | 5,8284 | 6,1944 |
| 1:1,47 | 0,11 | 11,00 | 6,2917 | |

Pour les concentrations les plus faibles en NaCl, aucun signal EEG n'a pu être détecté. Lorsque la concentration augmente, le signal devient détectable et le SNR augmente pour atteindre un optimum aux alentours de 5% de NaCl. Toutefois, lorsque l'on approche de la concentration de saturation, le SNR diminue. Il est supposé que cette diminution pourrait être due au fait que la présence d'une trop grande quantité d'ions entrave la mobilité de ceux-ci. Le SNR augmente à nouveau pour des concentrations sursaturées. Ceci pourrait s'expliquer par le dépôt de sels sur la surface des électrodes après séchage, ce qui augmenterait la conductivité. Toutefois, à ces fortes concentrations en NaCl, on observe également une détérioration de la surface des électrodes qui prennent un aspect huileux et sont impropres à l'utilisation.

Des essais ont également été effectués avec les composés polymériques chargés de poudre de graphite, pour évaluer l'influence de la charge en graphite.

Les résultats sont illustrés dans le Tableau 3 ci-dessous. Comme précédemment, les % de NaCl indiqués sont des pourcentages en poids par rapport au glycérol.

[Table 3]

| Graphite (g) | PVA:Glycérol:Graphite | [NaCl] (% p.p.) | *SNR* |
|---|---|---|---|
| 0,12 | 1 : 1,52 : 0,18 | 5,83 | / |
| 1,01 | 1 : 1,50 : 0,51 | 5,67 | 8,5763 |

Pour la quantité de graphite la plus faible (18% en poids du PVA) on ne détecte aucun signal. Toutefois, pour une quantité plus importante, on observe une augmentation significative du SNR.

**2) Mesure de la conductivité ionique**

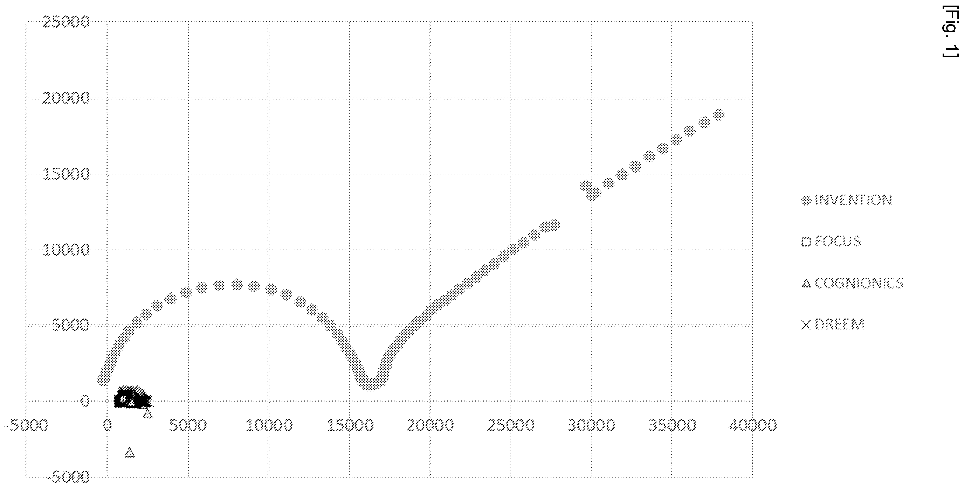

Les propriétés de conductivité ionique d'une électrode de l'invention (Rapport PVA :glycérol = 1:1,52 ; % en poids de NaCl par rapport au glycérol = 5 %) ont été comparées avec celles d'électrodes sèches de l'art antérieur : FOCUS Dry Active EEG Electrodes (TRANSCRANIAL) ; Flex Sensor (COGNIONICS) ; Électrode DREEM (DREEM).
L'impédance de chacune des électrodes a été mesurée à l'aide d'un multimètre Analog Discovery 2 (DIGILENT), et les résultats représentés sous forme de diagramme de Nyquist.
Les résultats sont illustrés par la Figure 1.
Légende de la Figure 1 : Axe des abscisses : partie réelle d'impédance Z' (en ohms) ; axe des ordonnées : partie imaginaire d'impédance Z" (en ohms) ; ●: électrode de l'invention ; △ : électrode COGNIONICS ; × : électrode DREEM ; □ : électrode FOCUS.
Dans le cas de l'électrode de l'invention, le tracé du diagramme est formé par un demi-cercle et une droite. Le demi-cercle représente une relaxation due au mouvement des ions à hautes fréquences et la droite à basse fréquence représente la polarisation aux électrodes. Ce tracé confirme que cette électrode est un conducteur ionique.
Dans le cas des électrodes de l'art antérieur, le tracé du diagramme montre principalement des amas de points regroupés sur l'axe des abscisses, et on n'observe pas le demi-cercle représentant le mouvement des ions. Ceci indique que les matériaux de ces électrodes sont des conducteurs électroniques mais ne sont pas des conducteurs ioniques.

**Revendications**

1. Composition polymérique conductrice ionique définie par la formule générale suivante :

$$(PH)x + (SOH)y + z(MCl) \, ;$$

dans laquelle :

- PH représente un polymère contenant des fonctions protiques constituées par des groupes hydroxyle ;
- SOH représente un polyol plastifiant de masse moléculaire supérieure ou égale à 75 g/mol et inférieure ou égale à 250 g/mol, sous forme de molécules discrètes ;
- MCl représente du chlorure de sodium ou de potassium (M= Na ou K) ;
- $0,3 \leq x/y \leq 3$, x représentant la quantité en poids du polymère PH, et y la quantité en poids du polyol SOH ;
- $0,5 \% \leq z \leq 15\%$, z représentant le pourcentage en poids de MCl par rapport au polyol SOH.

2. Composition polymérique selon la revendication 1, **caractérisée en ce que** le polymère PH est un alcool poly(vinylique) de degré de saponification supérieur ou égal à 60% et inférieur ou égal à 100%, et de masse moléculaire moyenne $M_w$ supérieure ou égale à $5.10^4$.

3. Composition polymérique selon l'une des revendications 1 ou 2, **caractérisée en ce que** le polyol plastifiant SOH est choisi parmi le glycérol, le propylène glycol, le dipropylène glycol ou leurs mélanges.

4. Composition polymérique selon l'une des revendications 1 à 3, **caractérisée en ce que** le rapport x/y est tel que $0,50 \leq x/y \leq 1,00$.

5. Composition polymérique selon l'une des revendications 1 à 4, **caractérisée en ce que** le pourcentage z est 1 %

$\leq z \leq 15$ %.

6. Composition polymérique selon l'une des revendications 1 à 5, **caractérisée en ce qu'**elle comprend en outre une charge carbonée particulaire électro-conductrice, et **en ce que** le pourcentage en poids de ladite charge conductrice par rapport au polymère PH est de 20% à 50%.

7. Composition polymérique selon la revendication 6, **caractérisée en ce qu'**elle comprend en outre un couple rédox permettant la transition de la conductivité ionique à la conductivité électronique.

8. Électrode de mesure d'un signal électro-physiologique comprenant une composition polymérique selon l'une des revendications 1 à 7.

9. Dispositif de mesure d'un signal électro-physiologique comprenant une ou plusieurs électrodes selon la revendication 8.

**Patentansprüche**

1. Polymerische Verbindung, die ionisch leitet, definiert durch die folgende allgemeine Formel:

$$(PH)x + (SOH)y + z(MCI);$$

wobei:

- PH für ein Polymer steht, das protische Funktionen enthält, die von Hydroxylgruppen konstituiert sind;
- SOH für ein plastifizierendes Polyol steht mit einer molekularen Masse größer als oder gleich 75 g/mol und geringer als oder gleich 250 g/mol in Form von diskreten Molekülen;
- MCI für Natrium- oder Kaliumchlorid steht (M = Na oder K) ;
- $0,3 \leq x/y \leq 3$, wobei x für die Menge nach Gewicht des Polymers PH steht und y für die Menge nach Gewicht des Polyols SOH steht;
- $0,5$ % $\leq z \leq 15$ %, wobei z für den Prozentsatz nach Gewicht von MCI im Vergleich zu dem Polyol SOH steht.

2. Polymerische Verbindung nach Anspruch 1, **dadurch gekennzeichnet, dass** das Polyol PH ein Poly(vinyl)alkohol mit einem Verseifungsgrad höher als oder gleich 60 % und geringer als oder gleich 100 % und mit einem mittleren Molekulargewicht $M_W$ höher als oder gleich $5.10^4$ ist.

3. Polymerische Verbindung nach einem der Ansprüche 1 oder 2, **dadurch gekennzeichnet, dass** das plastifizierende Polyol SOH ausgewählt ist aus Glycol, Glycoldipropylen oder deren Gemischen.

4. Polymerische Verbindung nach einem der Ansprüche 1 bis 3, **dadurch gekennzeichnet, dass** das Verhältnis x/y solcherart beschaffen ist, dass $0,50 \leq x/y \leq 1,00$.

5. 25. Polymerische Verbindung nach einem der Ansprüche 1 bis 4, **dadurch gekennzeichnet, dass** der Prozentsatz $1$ % $\leq z \leq 15$ % beträgt.

6. Polymerische Verbindung nach einem der Ansprüche 1 bis 5, **dadurch gekennzeichnet, dass** sie weiter eine partikuläre, elektrisch leitfähige Kohlenstoffladung umfasst, und dadurch, dass der Prozentsatz in Gewicht der leitfähigen Ladung im Verhältnis zu dem Polymer PH zwischen 20 % und 50 % beträgt.

7. Polymerische Verbindung nach Anspruch 6, **dadurch gekennzeichnet, dass** sie weiter ein Redoxpaar umfasst, das die Transition der ionischen Leitfähigkeit zu der elektronischer Leitfähigkeit ermöglicht.

8. Messelektrode für ein elektrophysiologisches Signal, die eine polymerische Verbindung nach einem der Ansprüche 1 bis 7 umfasst.

9. Messvorrichtung für ein elektrophysiologisches Signal, die eine oder mehrere Elektroden nach Anspruch 8 umfasst.

**Claims**

1. Ionic conductive polymer composition defined by the following general formula:

$$(PH)x + (SOH)y + z(MCl):$$

wherein:

- PH represents a polymer containing protic functions formed by hydroxyl groups;
- SOH represents a plasticising polyol with a molecular mass greater than or equal to 75 g/mol and less than or equal to 250 g/mol, in the form of discrete molecules;
- MCl represents sodium or potassium chloride (M = Na or K);
- $0.3 \leq x/y \leq 3$, x representing the quantity by weight of the polymer PH, and y the quantity by weight of the polyol SOH;
- $0.5\% \leq z \leq 15\%$, z representing the percentage by weight of MCl with respect to the polyol SOH.

2. Polymer composition according to claim 1, **characterised in that** the polymer PH is a poly(vinyl alcohol with a saponification value greater than or equal to 60% and less than or equal to 100%, and a mean molecular mass $M_W$ greater than or equal to $5.10^4$.

3. Polymer composition according to one of claims 1 or 2, **characterised in that** the plasticising polyol SOH is selected from glycerol, propylene glycol, dipropylene glycol or mixtures thereof.

4. Polymer composition according to one of claims 1 to 3, **characterised in that** the ratio x/y is such that $0.50 \leq x/y \leq 1.00$.

5. Polymer composition according to one of claims 1 to 4, **characterised in that** the percentage z is $1\% \leq z \leq 15\%$.

6. Polymer composition according to one of claims 1 to 5, **characterised in that** it furthermore comprises an electroconductive particulate carbonaceous filler, and **in that** the percentage by weight of said conductive filler with respect to the polymer PH is 20% to 50%.

7. Polymer composition according to claim 6, **characterised in that** it furthermore comprises a redox couple allowing transition from ion conductivity to electron conductivity.

8. Electrode for measuring an electrophysiological signal comprising a polymer composition according to one of claims 1 to 7.

9. Device for measuring an electrophysiological signal comprising one or more electrodes according to claim 8.

[Fig. 1]

## RÉFÉRENCES CITÉES DANS LA DESCRIPTION

**Documents brevets cités dans la description**

- FR 2464077

**Littérature non-brevet citée dans la description**

- **LOPEZ-GORDO et al.** *Sensors,* 2014, vol. 14 (7), 12847-12870